# EUROPEAN PATENT APPLICATION

(11) **EP 4 806 375 A1**
(43) Date of publication of application: **16.09.2026**
(21) Application number: 26155930.6
(22) Date of filing: 03.02.2026
(51) Int. Cl.: A61B 18/14, A61B 34/37, A61B 34/00

(54) **ROBOTIC SURGICAL SYSTEM AND METHOD FOR CONTROLLING ROBOTIC SURGICAL SYSTEM**

(30) Priority: 13.03.2025 JP 2025040439
(71) Applicant: Medicaroid Corporation, Kobe-shi, Hyogo 650-0047 (JP)
(72) Inventor: Shinagawa, Hiroki, Hyogo, 650-0047 (JP); Ota, Shinichi, Hyogo, 650-0047 (JP)
(74) Representative: Müller Hoffmann & Partner

(57) **Abstract**

In a robotic surgical system (500), one or more control devices (300, 310) control an amount of movement of an actuator (SM2) so that a gripper (211a, 211b) grips an object in response to a gripping input received by a first input device (111) including a pair of grip members (111a) that are able to be opened and closed and limit the amount of movement of the actuator for closing the gripper corresponding to the gripping input to a predetermined threshold (TH1) or less when a cutting procedure input is received by a second input device.

## Description

### TECHNICAL FIELD

The disclosure may relate to a robotic surgical system and a control method of a robotic surgical system.

### BACKGROUND

In a related art, there has been a robotic surgical system. Patent Document 1 (Japanese Patent No. 6141289) discloses a robotic surgery system including a patient cart and a surgeon console. The patient cart includes an articulating arm on which an electrosurgical instrument including an end effector for gripping a target is mounted. The surgeon console includes a master grip input mechanism for providing a grip input to open and close the end effector, and a foot pedal for providing a procedure input to perform a sealing procedure by the electrosurgical instrument as a surgical procedure. In this robotic surgical system, the grip force of the end effector is increased when a signal corresponding to the grip input by the master grip input mechanism reaches a threshold level and a signal of the procedure input by the foot pedal is in an on state.

Patent Document 1: Japanese Patent No. 6141289

### SUMMARY

In the robotic surgical system disclosed in Patent Document 1, the gripping force of the end effector is increased, but when thin and soft tissue is gripped with the end effector of the electrosurgical instrument and electrical energy for cutting is supplied, a short circuit may occur in the end effector due to the strong gripping force of the end effector. If the short circuit occurs in the end effector, while electricity flows within the end effector, electricity does not flow to the tissue on which the surgical procedure with the electrosurgical instrument is to be performed, so that the surgical procedure with the electrosurgical instrument may not be performed properly. Therefore, there is a need for a robotic surgical system and a controlling method of the robotic surgical system that can prevent short circuits caused by the strong gripping force of the end effector (gripper) and properly perform cutting as the surgical procedure with a bipolar electrosurgical instrument.

An object of an embodiment of the disclosure may be to provide a robotic surgical system and a control method for the robotic surgical system that are capable of preventing the short circuit caused by the strong gripping force of the gripper and properly performing cutting as the surgical procedure using bipolar electrosurgical instruments.

A first aspect of the disclosure may be a robotic surgical system, that may include a bipolar electrosurgical instrument including a gripper that is able to be opened and closed to grip an object; a robot arm to which the electrosurgical instrument is detachably attached and which includes an actuator configured to drive the gripper of the electrosurgical instrument; a first input device including a pair of grip members that are able to be opened and closed and receiving a gripping input instructing gripping by the gripper; a second input device that receives a cutting procedure input instructing to perform cutting as a surgical procedure by the electrosurgical instrument; and one or more control devices configured to control an amount of movement of the actuator so that the gripper closes in response to the gripping input received by the first input device, wherein the one or more control devices are configured to limit the amount of movement of the actuator for closing the gripper corresponding to the gripping input to a predetermined threshold or less when the cutting procedure input is received by the second input device.

According to the first aspect, as described above, the one or more control devices is configured to limit the amount of movement of the actuator for closing the gripper corresponding to the gripping input to the predetermined threshold or less when the cutting procedure input is received by the second input device. This allows the amount of movement of the actuator to be limited to the predetermined threshold or less when electrical energy is supplied to the electrosurgical instrument, thereby reducing the gripping force of the gripper. As a result, even when a thin and soft object is gripped by the gripper of the electrosurgical instrument, it is possible to prevent a short circuit from occurring due to a strong gripping force. This allows electricity to be appropriately applied to the object on which the cutting as the surgical procedure is to be performed with the electrosurgical instrument, so that the cutting with the electrosurgical instrument can be performed appropriately.

A second aspect of the disclosure may be a computer-implemented method for controlling a robotic surgical system, the method including: receiving, via a first input device provided in the robotic surgical system and including a pair of grip members that are able to be opened and closed, a gripping input instructing to grip the object by a gripper that is included in a bipolar electrosurgical instrument and is able to be opened and closed to grip an object; in response to receiving the gripping input, controlling an amount of movement of an actuator configured to drive the gripper so that the gripper closes, the actuator provided in a robot arm to which the electrosurgical instrument is detachably attached; receiving, via a second input device provided in the robotic surgical system, a cutting procedure input instructing to perform cutting as a surgical procedure by the electrosurgical instrument; and in response to receiving the cutting procedure input, limiting the amount of movement of the actuator for closing the gripper corresponding to the gripping input to a predetermined threshold or less.

According to the second aspect, as described above, the method includes in response to receiving the cutting procedure input, limiting an amount of movement of the actuator for closing the gripper corresponding to the gripping input to a predetermined threshold or less. This allows the amount of movement of the actuator to be limited to the predetermined threshold or less when electrical energy is supplied to the electrosurgical instrument, thereby reducing the gripping force of the gripper. As a result, even when a thin and soft object is gripped by the gripper of the electrosurgical instrument, it is possible to prevent a short circuit from occurring due to a strong gripping force. This allows electricity to be appropriately applied to the object on which the cutting as surgical procedure is to be performed with the electrosurgical instrument, so that the cutting with the electrosurgical instrument can be performed appropriately.

According to the aspects, it is possible to suppress the occurrence of a short circuit caused by the strong gripping force of the gripper, and to appropriately perform the cutting as the surgical procedure using the bipolar electrosurgical instrument.

### BRIEF DESCRIPTION OF DRAWINGS

FIG. 1 is a diagram illustrating a configuration of a robotic surgical system according to an embodiment;
FIG. 2 is a diagram illustrating a display of a medical cart according to the one embodiment;
FIG. 3 is a diagram illustrating a configuration of the medical cart according to the one embodiment;
FIG. 4 is a diagram illustrating a configuration of a robot arm according to the one embodiment;
FIG. 5 is a diagram showing a forceps;
FIG. 6 is a perspective diagram illustrating an electrosurgical instrument according to the one embodiment.;
FIG. 7 is a diagram illustrating an operation handle according to the one embodiment;
FIG. 8 is a perspective diagram illustrating a foot pedal according to the one embodiment;
FIG. 9 is a control block diagram of a robotic surgical system according to the one embodiment;
FIG. 10 is a control block diagram of a surgical robot according to the one embodiment;
FIG. 11 is a control block diagram of a positioner and the medical cart according to the one embodiment;
FIG. 12 is a diagram illustrating the configuration of an instrument and an electrosurgical unit according to the one embodiment;
FIG. 13 is a diagram for explaining a relationship between opening and closing angles of an operation handle and opening and closing angles of an end effector according to the one embodiment;
FIG. 14 is a graph showing a relationship between opening and closing angles of the operation handle and opening and closing angles of the end effector according to the one embodiment;
FIG. 15 is a diagram (1) for explaining limitation of a motor movement amount according to the one embodiment;
FIG. 16 is a diagram (2) for explaining limitation of a motor movement amount according to the one embodiment;
FIG. 17 is a graph for explaining a time change in a motor movement amount command value and an actual motor movement amount when the motor movement amount is reduced to a predetermined threshold or less when the end effector exhibits unnatural movement;
FIG. 18 is a graph for explaining the time change in the motor movement amount command value and the actual motor movement amount when the motor movement amount is reduced to a predetermined threshold or less according to the one embodiment;
FIG. 19 is a diagram for explaining an electrosurgical instrument and a first controller according to a modified embodiment;
FIG. 20 is a diagram for explaining a robot arm and a first controller according to a modified embodiment.

### DETAILED DESCRIPTION

### (Configuration of Robotic Surgical System)

The following description describes a configuration of a robotic surgical system 500 according to this embodiment. The robotic surgical system 500 includes a surgical robot 100, a remote operation apparatus 200, a third controller 300 and an image processing unit 400.

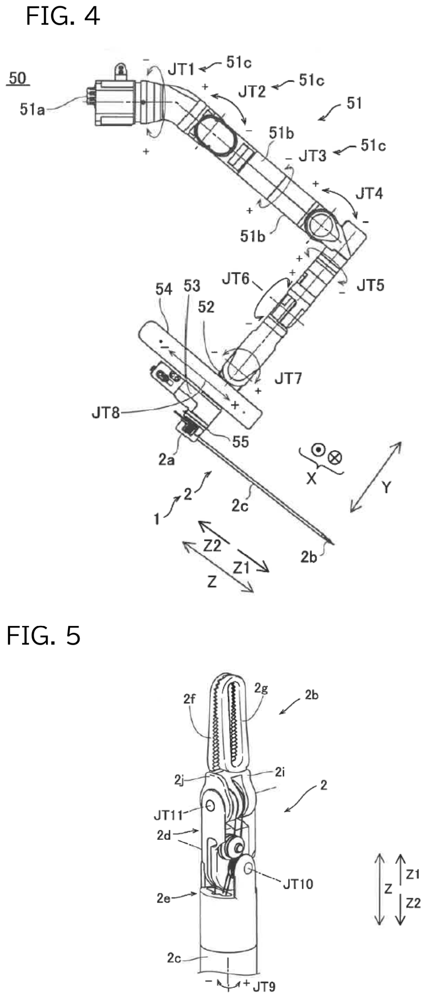

In this specification, a longitudinal direction of an instrument 1 is defined as a Z direction as shown in FIG. 4. A fore-end side of the instrument 1 is defined as a Z1 side, and a base-end side of the instrument 1 is defined as a Z2 side. A direction orthogonal to the Z direction is defined as an X direction. A direction orthogonal to the Z direction and the X direction is defined as a Y direction.

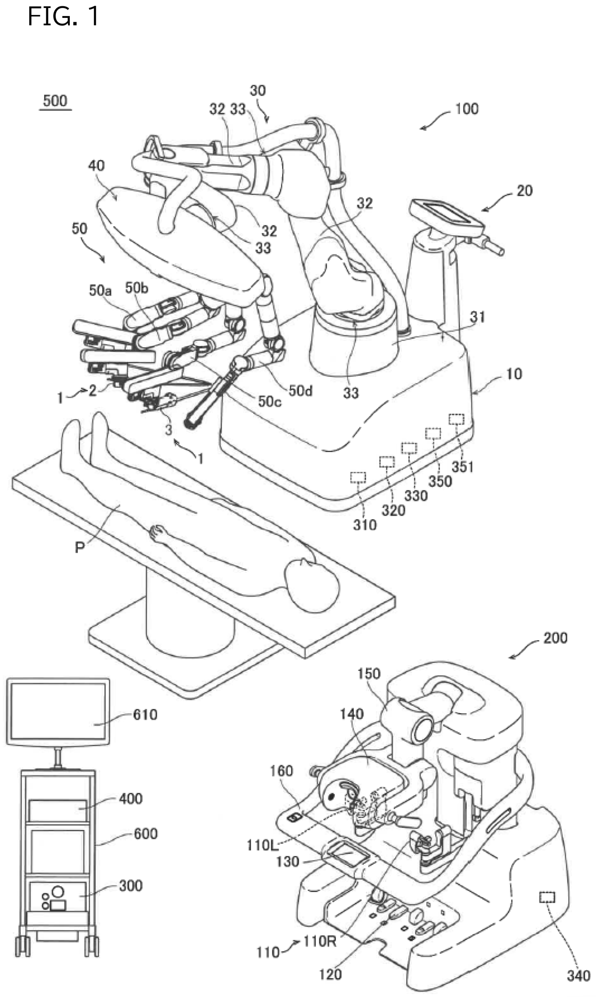

As shown in FIG. 1, the surgical robot 100 is arranged in an operating room. The remote operation apparatus 200 is located remote from the surgical robot 100. Also, the remote operation apparatus 200 is configured to receive operations as to the instruments 1. Specifically, an operator, such as a doctor, can provide the remote operation apparatus 200 with an instruction to instruct a desired motion of the surgical robot 100. The remote operation apparatus 200 transmits the provided command to the surgical robot 100. The surgical robot 100 is configured to perform the motion in accordance with the command received. The surgical robot 100 is arranged in the operating room, which is a sterile field.

### (Configuration of Surgical Robot)

As shown in FIG. 1, the surgical robot 100 includes a medical cart 10, a cart positioner operation unit 20, a positioner 30, an arm base 40 and a plurality of robot arms 50.

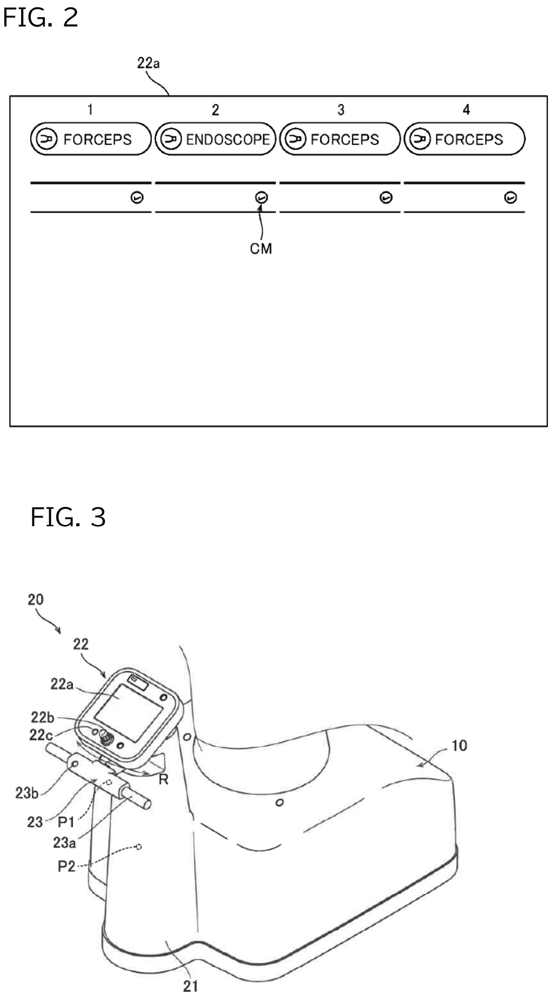

As shown in FIG. 3, the cart positioner operation unit 20 is arranged in a rear part of the medical cart 10 and supported by a cart positioner operation support 21, and the medical cart 10 or the positioner 30 can be moved by operating the operating handle 23 of the cart positioner operation unit 20. The cart positioner operation unit 20 includes an input device 22 and an operation handle 23. The input device 22 is configured to accept operations to move or change orientations of the positioner 30, the arm base 40 and the plurality of robot arms 50 to prepare a surgical operation mainly before the operation is carried out.

As shown in FIG. 3, the input device 22 includes the display 22a, a joystick 22b and an enable switch 22c. For example, the display 22a is a liquid crystal touch panel. As shown in FIG. 2, the display 22a indicates numbers corresponding to the plurality of robot arms 50. Also, the display 22a indicates types of surgical instruments 2 attached to the plurality of robot arms 50. The display 22a displays a check mark CM indicating that a pivot position serving as a fulcrum for moving the surgical instrument 2 and the endoscope 3 has been set.

As shown in FIG. 3, the joystick 22b is arranged in proximity to the display 22a of the input device 22. When an operation mode displayed on the input device 22 is selected, the positioner 30 can be three-dimensionally moved by operating the joystick 22b..

The enable switch 22c is arranged in proximity to the joystick 22b. The enable switch 22c is configured to enable or disable movement of the positioner 30. When the enable switch 22c is pressed so that movement of the positioner 30 is enabled, the positioner 30 can be moved in accordance with a manual operation of the joystick 22b.

Also, the operation handle 23 is arranged in proximity to the display 22a of the medical cart 10. The operating handle 23 includes a throttle 23a that is configured to be gripped and twisted by an operator such as nurse, engineer, etc. to control movement of the medical cart 10. Specifically, the operation handle 23 is arranged under the input device 22. The operation handle 23 is configured to be rotatable in the right and left directions shown in the R direction, and the medical cart 10 rotates with the rotation of the operation handle 23.

Also, the operation handle 23 includes an enable switch 23b configured to enable or disable movement of the medical cart 10. While the enable switch 23b is pressed so that movement of the medical cart 10 is enabled, the medical cart 10 can be moved in accordance with a manual operation of the throttle 23a of the operating handle 23.

For example, as shown in FIG. 1, the positioner 30 is constructed of a 7-axis multi-joint robot. The positioner 30 is arranged on the medical cart 10. The positioner 30 is configured to adjust a position of the arm base 40. The positioner 30 can three-dimensionally move the position of the arm base 40.

The positioner 30 includes a base 31, and a plurality of links 32 coupled to the base 31. The links 32 are coupled to each other by joints 33.

The arm base 40 is attached to the distal end of the positioner 30. The base ends of the plurality of robot arms 50 are attached to the arm base 40. The plurality of robot arms 50 are foldable into a storage posture. The arm base 40 and the plurality of robot arms 50 covered by sterile drapes when used. The robot arms 50 respectively support the instrument 1.

Two or more robot arms 50 are provided as a plurality of robot arms 50. Specifically, four robot arms 50a, 50b, 50c and 50d are provided. The robot arms 50a, 50b, 50c and 50d have a similar configuration to each other.

As shown in FIG. 4, the robot arm 50 includes an arm portion 51, a first link 52, a second link 53, and a translation mechanism 54. The robot arm 50 includes JT1, JT2, JT3, JT4, JT5, JT6 and JT7 axes as rotational axes and JT8 axes as linear axes. The axis from JT1 to JT7 is the axis of rotation of the joints 51c of the arm portion 51. The JT7 axis is a rotation axis of the first link 52. The JT8 axis line is an axis line of a linear motion joint in which the translation mechanism 54 moves the second link 53 relative to the first link 52 in the Z direction. The arm portion 51 includes a base portion 51a, link portions 51b, and the joints 51c.

The arm portion 51 is constructed of a 7-axis multi-joint robot arm. The first link 52 is arranged in a distal end of arm portion 51. The translation mechanism 54 is arranged between the first link 52 and the second link 53. The second link 53 includes a holder 55 configured to hold the instrument 1. The translation mechanism 54 is configured to translationally move the holder 55 to which the instrument 1 is attached between a first position and a second position. The first position is a position of a Z2-direction side end of a moving range of the holder 55 moved by the translation mechanism 54 along the linear axis JT8. The second position is a position of a Z1-direction side end of the moving range of the holder 55 moved by the translation mechanism 54 along the linear axis JT8.

Instruments 1 can be attached to the distal ends of the plurality of robot arms 50. The instruments 1 include, for example, replaceable surgical instruments 2, and an endoscope 3 configured to capture images of a part to be operated. As shown in FIG. 4, the surgical instrument 2 includes a driven unit 2a, a forceps 2b and a shaft 2c.

As shown in FIG. 1, an endoscope 3 is attached to the distal end of one, e.g., the robot arm 50c of the robot arms 50, and the surgical instruments 2 are attached to the distal ends of the others, e.g., the robot arms 50a, 50b and 50d. The endoscope 3 is attached to one of two robot arms 50b and 50c, which are located in a central part, of the four robot arms 50 arranged adjacent to each other.

### (Configuration of Instrument)

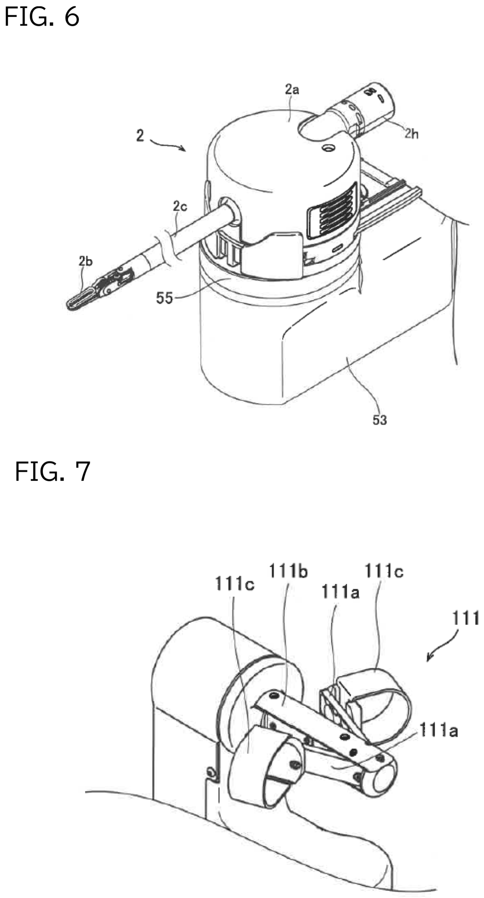

For example, as shown in FIG. 5, a forceps 2b is provided to the distal end of the surgical instrument 2. The forceps 2b includes a first support 2d and a second support 2e. The first support 2d is configured to rotatably support a base end side of jaws 2f and 2g about an axis JT11. The second support 2e is rotatably configured to support a proximal end of the first support 2d about an axis JT10. The shaft 2c can rotate about an axis JT9. The jaws 2f and 2g can pivot about the axis JT11 to open and close. As shown in FIG. 6, the surgical instrument 2, which is a bipolar electrosurgical instrument, is provided with a connector 2h. An electric wiring L1a from an electrosurgical unit 220, which will be described later, is connected to the connector 2h. Each of electric wires is connected to the base end of each of the jaw members 2g and 2f. The electric wires at the base ends of each of the jaw members 2g and 2f are connected to the connector 2h, and electric energy for coagulation or electric energy for cutting is supplied from the electrosurgical unit 220 to the jaw members 2g and 2f via the wiring L1a. As shown in FIG. 5, the jaw member 2g and the jaw member 2f are supported on the first support 2d in a state insulated by a plastic pulley 2i connected to the base end of the jaw member 2g and a plastic pulley 2j connected to the base end of the jaw member 2f.

### (Remote Operation Apparatus)

As shown in FIG. 1, the remote operation apparatus 200 is arranged in an operating room or outside the operating room. The remote operation apparatus 200 includes operation units 110, foot pedals 120, a touch panel 130, a monitor 140, a support arm 150, and a support bar 160. Each of the operation units 110 serves as a handle for operation that is configured to receive commands from an operator such as doctor.

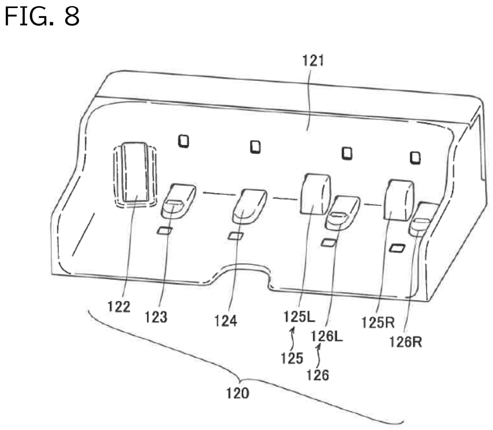

The operation units 110 are handles configured to manipulate the surgical instrument 2. Also, the operation units 110 are configured to receive operations as to the surgical instruments 2. The operation units 110 include an operation manipulator 110L that is arranged on a left side from viewpoint of an operator such as doctor and is configured to be manually operated by a left hand of the operator, and an operation manipulator 110R that is arranged on a right side from viewpoint of the operator and is configured to be manually operated by a right hand of the operator. The operation manipulator 110 L and the operation manipulator 110R operate the robot arm 50, respectively. For example, the endoscope 3 is attached to one of two robot arms 50 arranged in the center among the four robot arms 50. For example, as shown in FIG. 1, when the endoscope 3 is attached to the robot arm 50c, the operation manipulator 110 L operates the robot arm 50a or 50b, and the operation manipulator 110R operates the robot arm 50d. When the endoscope 3 is attached to the robot arm 50b, the operation manipulator 110 L operates the robot arm 50a, and the operation manipulator 110R operates the robot arm 50c or 50d. The robot arm 50b or 50c to which the endoscope 3 is attached is operated by the operation manipulator 110 L and the operation manipulator 110R while the camera pedal 124 shown in FIG. 8 is depressed.

As shown in FIG. 7, each of the operation manipulators 110R and 110L includes an operating handle 111. The operating handle 111 includes a pair of grip members 111a that are opened and closed by the operator. The grip members 111a are made of elongated plate-like lever members, and the proximal ends of the pair of grip members 111a are rotatably connected to the proximal end of the grip support member 111b. Cylindrical finger insertion portions 111c are arranged on the grip members 111a. The operator inserts his fingers into the pair of finger insertion portions 111c to operate the operating handle 111. The opening and closing angle between the jaw members 2g and 2f is changed by increasing or decreasing the opening and closing angle between the pair of grip members 111a. A magnet is arranged on one side of the grip members 111a, and a Hall sensor is arranged on the grip support member 111b. When the operator opens and closes the grip member 111a, the magnet and Hall sensor function as an angle detection sensor, and the Hall sensor outputs the detected angle. Note that as the angle detection sensor, a Hall sensor may be placed in the grip member 111a and a magnet in the grip support member 111b. The pair of grip members 111a are an example of a "first input section".

As shown in FIG. 1, the monitor 140 is a scope-type display device configured to display images captured by the endoscope 3. The support arm 150 supports the monitor 140, and can adjust a height of the monitor 140 to a height of eyes of the operator such as doctor. The touch panel 130 is arranged on the support bar 160. When a head of the operator is detected by a sensor arranged in proximity to the monitor 140, the surgical robot 100 can accept manual operations from the remote operation apparatus 200. The operator will manually operate the operation unit 110 and the foot pedals 120 while seeing of an affected area on the monitor 140. These manual operations input instructions to the remote control apparatus 200. The instructions input to the remote control apparatus 200 is transmitted to the first controller 310 of the surgical robot 100.

As shown in FIG. 8, a plurality of foot pedals 120 configured to activate functions of the surgical instruments 2. The plurality of foot pedals 120 are provided in a base 121. The foot pedals 120 include a switching pedal 122, a clutch pedal 123, a camera pedal 124, incision pedals 125, coagulation pedals 126, and foot detectors 127. The switching pedal 122, the clutch pedal 123, the camera pedal 124, the incision pedals 125, the coagulation pedals 126 are configured to be operated by an operator's foot. Also, the incision pedals 125 include an incision pedal 125R corresponding to a right-side robot arm 50 and an incision pedal 125L corresponding to a left-side robot arm 50. Also, the coagulation pedals 126 include a coagulation pedal 126R corresponding to a right-side robot arm 50 and a coagulation pedal 126L corresponding to a left-side robot arm 50. Thus, since the operating handle 111, the coagulation pedal 126 and the cutting pedal 125 are assembled in the remote operation apparatus 200. Further, since the coagulating pedal 126R and the cutting pedal 125R are provided, the coagulating and cutting of the operation site can be performed by using the surgical instrument 2 operated by the right hand. Similarly, since the coagulating pedal 126 L and the cutting pedal 125 L are provided, the surgical instrument 2 operated with the left hand can be used to coagulate and cut the surgical site. The cutting pedal 125 is an example of a "second input device ".

The switching pedal 122 is configured to switch between the robot arms 50 to be operated by the operation unit 110. The clutch pedal 123 executes a clutch operation that temporarily disconnects the operational connection between the robot arm 50 and the operation unit 110. While the clutch pedal 123 is pressed by the operator, operations provided by the operation unit 110 is not transmitted to the robot arm 50. While the camera pedal 124 is pressed by the operator, the robot arm 50 that holds the endoscope 3 can be operated through the operation unit 110. While the cut pedal 125 or the coagulation pedal 126 is depressed by the operator, electrical energy is supplied from the electrosurgical unit 220 to the surgical instrument 2.

As shown in FIG. 1, a cart 600 holds a third controller 300 and an image processing unit 400. The image processing unit 400 is configured to process images captured by the endoscope 3. A display 610 is arranged on the cart 600. The display 610 is configured to display images captured by the endoscope 3.

### (Configuration of Control System)

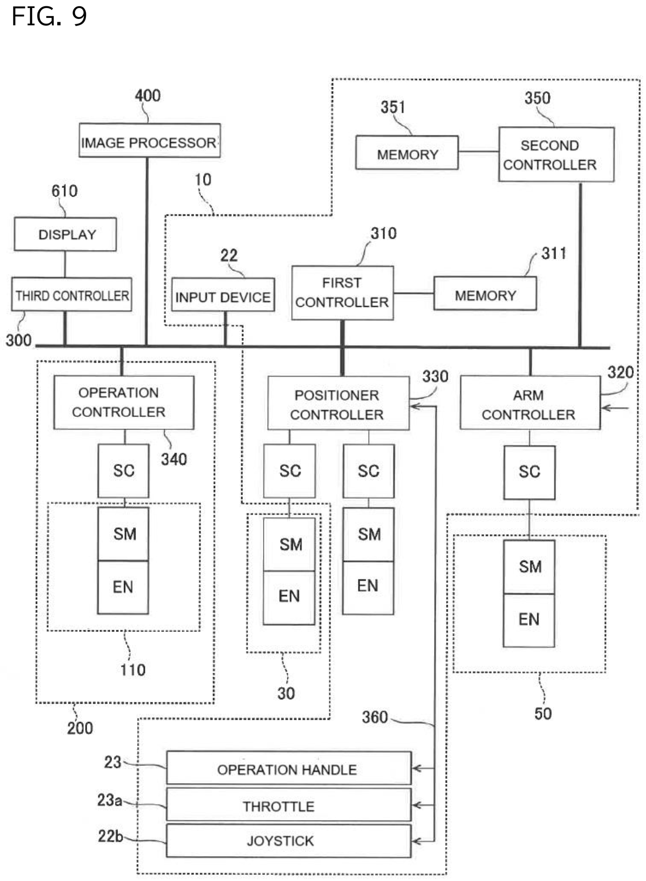

As shown in FIG. 9, the robotic surgical system 500 includes a first controller 310, an arm controller 320, a positioner controller 330, operation controllers 340 and a second controller 350. In addition, the robotic surgical system 500 includes a memory 311 connected to the first controller 310, a memory 351 connected to the second controller 350 and a third controller 300. As shown in FIG. 1, the second controller 350 and the memory 351 are arranged inside the medical cart 10, for example. The first controller 310 and the third controller 300 are examples of "one or more control devices".

The first controller 310 is accommodated in the medical cart 10, and configured to communicate with the arm controller 320 and the positioner controller 330 so that the robotic surgical system 500 is entirely controlled. Specifically, the first controller 310 is configured to control the arm controller 320, the positioner controller 330 and the operation controllers 340 by using the communications with them. The first controller 310 is connected to the arm controller 320, the positioner controller 330 and the operation controllers 340 through LAN, etc. The first controller 310 is arranged in the medical cart 10.

Each of the plurality of robot arms 50 includes the arm controller 320. In other words, a plurality of arm controllers 320 the number of which corresponds to the number of the plurality of robot arms 50 are included in the medical cart 10.

As shown in FIG. 9, the input device 22 is connected to the first controller 310 through LAN, etc. The operation handle 23, the throttle grip 23 a and the joystick 22 b are connected to the positioner controller 330 through a wiring line 360 by means of a communication network that can share information with them by using serial communication.

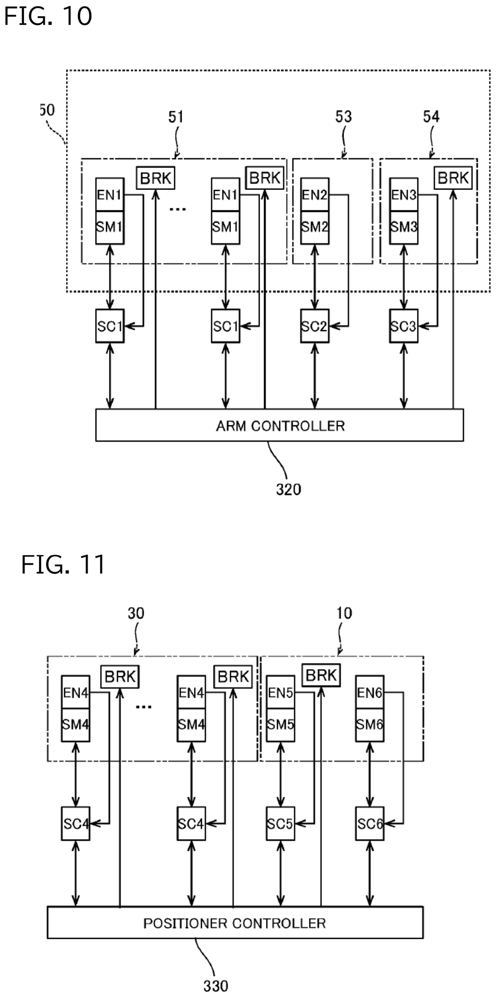

As shown in FIG. 10, each arm portion 51 includes a plurality of servomotors SM1, a plurality of encoders EN1 and a plurality of speed reducers corresponding to the Joints 51c. The encoder EN1 is configured to detect a rotation angle of the servomotor SM1. The speed reducer is configured to reduce a rotation of the servomotor SM1 whereby increasing its torque. A servo controller SC1 is configured to control the servomotor SM1, and is arranged in the medical cart 10 adjacent to the arm controller 320. Also, the encoder EN1 is configured to detect the rotation angle of the servomotor SM1, and is electrically connected to the servo controller SC1.

The second link 53 includes servomotors SM2 configured to rotate a driven member arranged in a driven unit 2a of the surgical instrument 2, encoders EN2, and speed reducer s. The encoder EN2 is configured to detect a rotation angle of the servomotor SM2. The speed reducer is configured to reduce a rotation of the servomotor SM2 whereby increasing its torque. The medical cart 10 includes a servo controller SC2 configured to control the servomotors SM2 for driving the surgical instrument 2. The encoder EN2 for detecting the rotation angle of the servo motor SM2 is electrically connected to the servo control unit SC2. Note that a plurality of servomotors SM2, a plurality of encoders EN2 and a plurality of servo controllers SC2 are included. The servo motor SM2 is an example of an "actuator".

The translation mechanism 54 includes a servomotor SM3 configured to translationally move the surgical instrument 2, an encoder EN3, and a speed reducer. The encoder EN3 is configured to detect a rotation angle of the servomotor SM3. The speed reducer is configured to reduce a rotation of the servomotor SM3 whereby increasing its torque. The medical cart 10 includes a servo controller SC3 configured to control the servomotor SM3 for translationally moving the surgical instrument 2. The encoder EN3 for detecting the rotation angle of the servo motor SM3 is electrically connected to the servo controller SC3.

The first controller 310 is configured to generate instruction values that specify positions of the servomotor SM1, SM2 and SM3 in accordance with manual operation that are received by the operation unit 110 and the operation handle 111 of the remote operation apparatus 200, and to drive the servomotor SM1, SM2 and SM3 in accordance with the instruction values.

As shown in FIG. 11, the positioner 30 includes a plurality of servomotors SM4, a plurality of encoders EN4 and a plurality of speed reducers corresponding to a plurality of joints 33 of the positioner 30. Each encoder EN4 is configured to detect a rotation angle of the servomotor SM4. The speed reducer is configured to reduce a rotation of the servomotor SM4 whereby increasing its torque.

The medical cart 10 includes wheels including front wheels as driving wheels, and rear wheels configured to be steered by manually operating the handle 23. The rear wheels are arranged closer to the operating handle 23 with respect to the front wheels. The medical cart 10 includes a servomotor SM5 configured to drive the front wheels of the medical cart 10, an encoder EN5, speed reducers, and brakes BRK. The speed reducer is configured to reduce a rotation of the servomotor SM5 whereby increasing its torque. Also, the operation handle 23 includes a potentiometer P1 shown in FIG. 3, and the servomotor SM5 of the front wheels can be driven in accordance with a rotation angle detected by the potentiometer P1 in response to a twisting amount of the throttle 23a. Also, the operation handle 23 of the medical cart 10 includes a potentiometer P2 shown in FIG. 3 on a turning shaft, and the rear wheel of medical cart 10 is provided with a servomotor SM6, an encoder EN6, and speed reducers. The speed reducer is configured to reduce a rotation of the servomotor SM6 whereby increasing its torque. The servomotor SM6 can be driven in accordance with a rotation angle detected by the potentiometer P2 in response to a rightward/leftward turning amount of the operation handle 23. In other words, power is assisted by the servomotor SM6 when the rear wheels are steered by turning the operation handle 23 rightward or leftward.

As shown in FIG. 11, the medical cart 10 includes servo controllers SC4 configured to control the servomotors SM4 for moving the positioner 30. Also, the encoder EN4 is configured to detect the rotation angle of the servomotor SM4, and is electrically connected to the servo controller SC4. The medical cart 10 includes a servo controller SC5 configured to control the servomotor SM5 for driving the front wheels of the medical cart 10. The encoder EN5 for detecting the rotation angle of the servo motor SM5 is electrically connected to the servo controller SC5. The medical cart 10 includes a servo controller SC6 configured to control the servomotor SM6 for power assistance to steering of the rear wheels of the medical cart 10. The encoder EN6 for detecting the rotation angle of the servo motor SM6 is electrically connected to the servo control unit SC6.

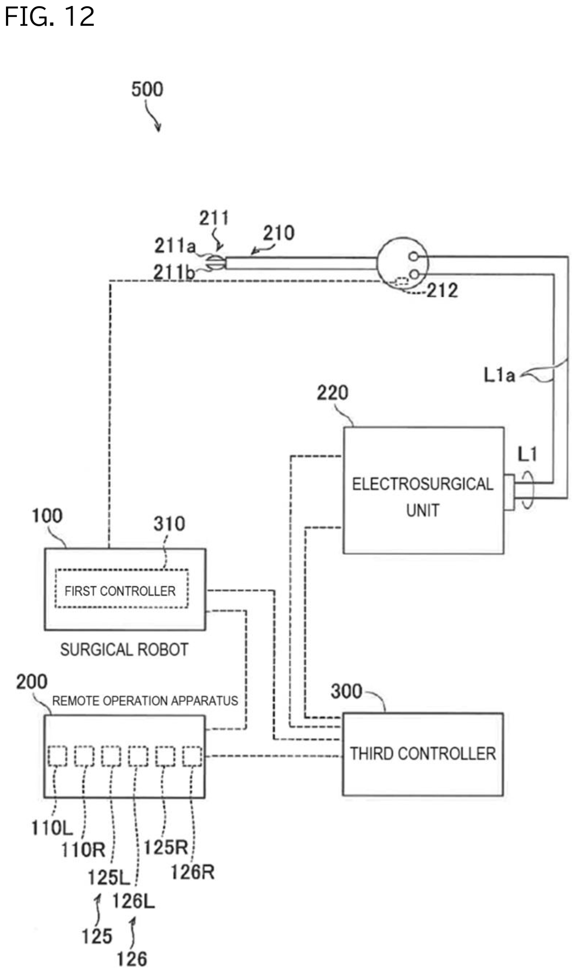

As shown in FIGS. 11 and 12, the Joints 51c of the arm portion 51, and the joints 33 of the positioner 30 includes their brake BRK. Also, the front wheels of the medical cart 10, the arm base 40 and the translation mechanism 54 includes their brake BRK. The arm controller 320 is configured to one-directionally transmit control signals to the brakes BRK of the Joints 51c of the arm portion 51, and the translation mechanism 54. The control signals are configured to indicate on/off of the brakes BRK.

The medical cart 10 includes the positioner controller 330. The positioner controller 330 is configured to control the positioner 30 and the medical cart 10.

The operation controllers 340 are provided in a main body of the remote operation apparatus 200. The operation controllers 340 are configured to control the operating units 110. The operation controllers 340 are arranged so as to correspond to each of the left-hand side operation manipulator 110L and the righthand side operation manipulator 110R. The operation unit 110 includes servomotors SM, encoders EN and speed reducers corresponding to the plurality of joints of the operation unit 110. The servo controllers SC configured to control the servomotors SM of the operation unit 110 is provided in the main body of the remote operation apparatus 200 adjacent to the operation controllers 340.

As shown in FIG. 9, the third controller 300 and the image processing unit 400 are connected to the first controller 310 through LAN. The display 610 is connected to the third controller 300.

### (Composition of Instruments and Electrosurgical Units)

As described above, the surgical instrument 2 is detachably attached to the robot arms 50a, 50b and 50d. A plurality of kinds of instruments 2 to be attached to the robot arm 50 are prepared. As shown in FIG. 12, an instrument 210 is a bipolar electrosurgical instrument, for example. The surgical instrument 210 includes an end effector 211 that can be opened and closed to grasp an object, and a memory 212 storing tool information of the surgical instrument 210. The end effector 211 is, as described above, e.g., forceps. The end effector 211 includes a first jaw member 211a and a second jaw member 211b that are able to be opened and closed by rotating relative to one another. The end effector 211 coagulates or cuts an object grasped between the first jaw member 211a and the second jaw member 211b by passing a high frequency current through the object. The end effector 211 may also include a second jaw member that can be opened and closed relative to the fixed first jaw member. The tool information of the surgical instrument 210 includes the type of instrument 210, information about the electrosurgical unit 220 that supplies electrical energy to the surgical instrument 210, etc. The surgical instrument 210 is an example of an "electrosurgical instrument", and the first jaw member 211a and the second jaw member 211b are an example of a "gripper".

The electrosurgical unit 220 supplies electrical energy to the surgical instrument 210. The electrical energy supplied by the electrosurgical unit 220 includes, for example, coagulating electrical energy for coagulating an object and cutting electrical energy for cutting the object. The first controller 310, the second controller 350 and the third controller 300 of the robotic surgical system 500 cannot receive the device information of the electrosurgical unit 220 from the electrosurgical unit 220. Specifically, the third controller 300 and the electrosurgical unit 220 are connected and it is possible for the third controller 300 to transmit ON and OFF signals to the electrosurgical unit 220, but the third controller 300 cannot receive device information from the electrosurgical unit 220.

When the surgical instrument 210 is attached to the robot arm 50 operated by the operation unit 110, the first controller 310 acquires tool information of the surgical instrument 210 from the memory 212. The first controller 310 determines whether to assign the coagulation pedal 126 and the cutting pedal 125 to the surgical instrument 210 based on the acquired tool information. When the surgical instrument 210 is an electrosurgical instrument, the first controller 310 assigns the coagulation pedal 126R and the cutting pedal 125R to the surgical instrument 210 attached to the robot arm 50 operated by the operation manipulator 110R, and assigns the coagulation pedal 126L and the cutting pedal 125L to the surgical instrument 210 attached to the robot arm 50 operated by the operation manipulator 110L. The coagulation pedal 126 and the cutting pedal 125 perform a procedure input to instruct the execution of a surgical procedure by the surgical instrument 210. Specifically, the coagulation pedal 126 provides the procedure input that instructs the performance of coagulation as a surgical procedure by the surgical instruments 210. The cut pedal 125 provides a treatment input that instructs the performance of cutting as a surgical procedure by the surgical instruments 210. The third controller 300 is configured to send a signal regarding electrical energy supply to the electrosurgical unit 220 while a treatment input is being performed by the coagulation pedal 126 or the cut pedal 125. Specifically, the third controller 300 is configured to send an ON signal to the electrosurgical unit 220.

As shown in FIG. 12, an electrical supply line L1 is provided for supplying bipolar electrical energy from the electrosurgical unit 220 to the surgical instrument 210. The supply line L1 can supply electrical energy for coagulation and electrical energy for cutting to the end effector 211. When a procedure input is performed by the coagulation pedal 126, the electrical energy for coagulation is supplied from the electrosurgical unit 220 to the surgical instrument 210 via the supply line L1. When a procedure input is performed by the cutting pedal 125, the electrical energy for cutting is supplied from the electrosurgical unit 220 to the surgical instrument 210 via the supply line L1. The surgical instrument 210 and the electrosurgical unit 220 are connected by a electrical wiring L1a.

### (Opening and closing control of end effector)

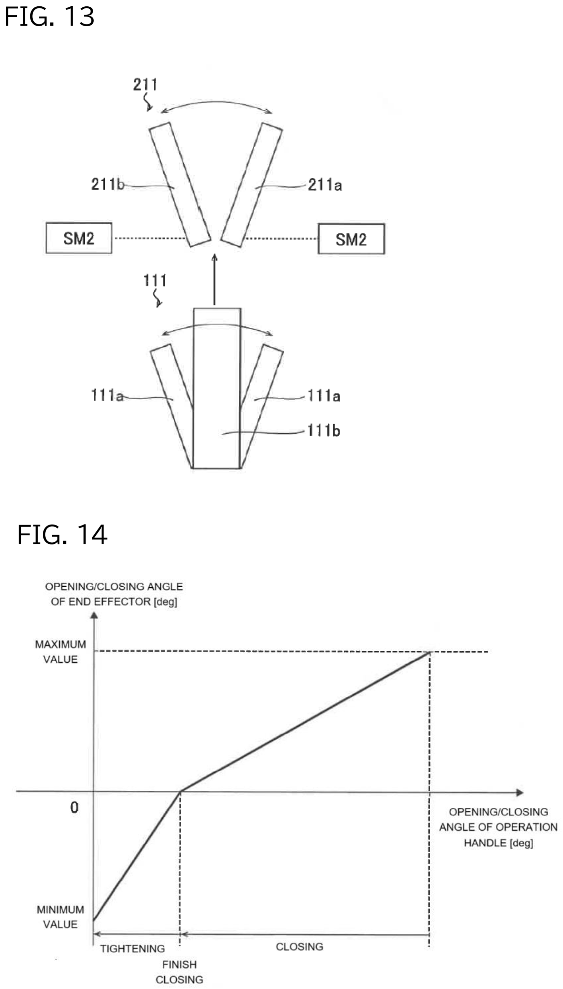

As shown in FIG. 13, the robot arm 50 includes two servo motors SM2 as the two actuators that drive the first jaw member 211a and the second jaw member 211b of the end effector 211 of the attached instrument 210. The pair of grip members 111a of the operation handle 111 are provided so as to be openable and closable relative to the grip support member 111b in order to perform the gripping input for opening and closing the first jaw member 211a and the second jaw member 211b of the end effector 211. The first controller 310 is configured to control the amount of movement of the servo motors SM2 so that the first jaw member 211a and the second jaw member 211b of the end effector 211 grip an object with a grip force corresponding to the gripping input by the operation handle 111. The amount of movement of the servo motor SM2 is the amount of rotation (angle of rotation or number of rotations) of the servo motor SM2. This makes it possible to easily control the grip force of the end effector 211 by effectively utilizing the amount of rotation of the servo motor SM2.

As shown in FIG. 14, the first controller 310 is configured to control the amount of movement of the servo motors SM2 based on a predetermined relationship between the opening/closing angle of the operation handle 111 and the opening/closing angle of the end effector 211. The first controller 310 is configured to generate a command value for the amount of movement each of the servo motors SM2 so that the opening/closing angle of the end effector 211 becomes a value corresponding to the opening/closing angle of the operation handle 111.

Figure 14 is a graph showing the relationship between the opening and closing angle of the operation handle 111 (a pair of grip members 111a) and the opening and closing angle of the end effector 211 (first jaw member 211a and second jaw member 211b). In the graph shown in Figure 14, the horizontal axis is the opening and closing angle of the operation handle 111, and the vertical axis is the opening and closing angle of the end effector 211. The graph shown in Figure 14 shows that as the opening and closing angle of the operation handle 111 becomes smaller, the opening and closing angle of the end effector 211 gradually becomes smaller, and that after the end effector 211 is completely closed (i.e., after the opening and closing angle becomes zero), the servo motors SM2 rotate to move the end effector 211 further in the closing direction, causing the end effector 211 to generate a tightening force (a gripping force).

### (Control to limit the amount of movement of the servo motor)

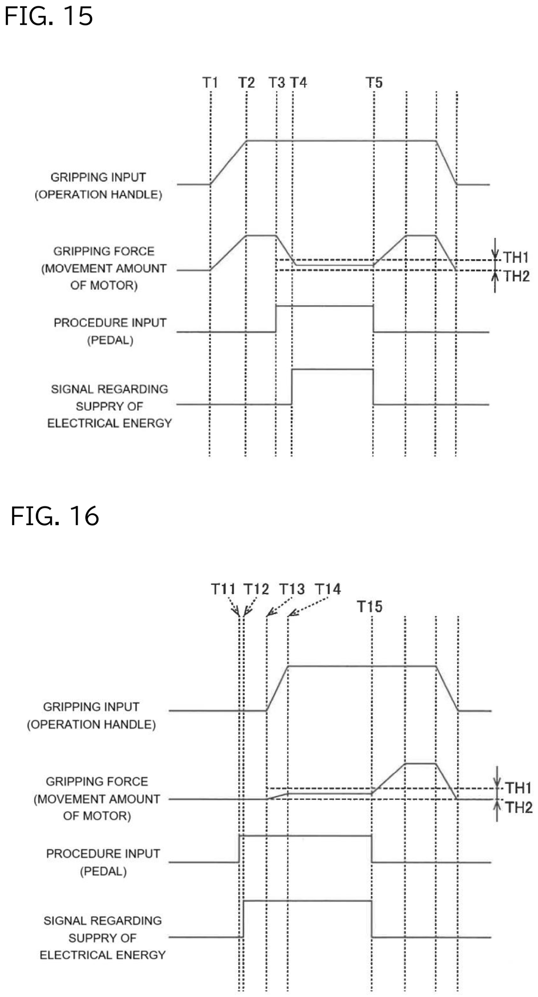

In this embodiment, as shown in FIG. 15, when the procedure input is performed by the cutting pedal 125, the first controller 310 is configured to limit the amount of movement of the servo motors SM2 to a predetermined threshold value TH1 or less in order to reduce the gripping force of the first jaw member 211a and the second jaw member 211b of the end effector 211 due to the gripping input. When the gripping input is being performed, the third controller 300 is configured to transmit a signal (ON signal) regarding electrical energy supply to the electrosurgical unit 220 based on the amount of movement of the servo motors SM2 becoming equal to or less than the predetermined threshold value TH1. While receiving the signal (ON signal) regarding electrical energy supply, the electrosurgical unit 220 supplies electrical energy for cutting to the surgical instrument 210. This allows the amount of movement of the servo motors SM2 to be limited to a predetermined threshold value TH1 or less to reduce the gripping force of the first jaw member 211a and the second jaw member 211b of the end effector 211 before electrical energy is supplied to the surgical instrument 210. Also, even when a thin and soft object is gripped and cut by the first jaw member 211a and the second jaw member 211b of the end effector 211, it is possible to prevent a short circuit from occurring between the first jaw member 211a and the second jaw member 211b due to a strong gripping force. This allows electricity to be properly applied to the object on which the surgical procedure is to be performed by the surgical instrument 210, so that the surgical procedure by the surgical instrument 210 can be properly performed. As a result, it is possible to prevent a short circuit from occurring due to a strong gripping force of the end effector 211, and it is possible to properly perform the surgical procedure by the surgical instrument 210.

Also, in this embodiment, the first controller 310 is configured to limit the amount of movement of the servo motors SM2 to the predetermined threshold value TH1 or less by changing the relationship between the opening/closing angle of the operation handle 111 (a pair of grip members 111a) and the opening/closing angle of the end effector 211 (first jaw member 211a and second jaw member 211b). As a result, the amount of movement of the servo motors SM2 can be easily limited to a predetermined threshold value TH1 or less simply by changing the relationship between the opening/closing angle of the operation handle 111 and the opening/closing angle of the end effector 211. The first controller 310 is configured to change the relationship between the opening/closing angle of the operation handle 111 and the opening/closing angle of the end effector 211 based on the procedure input being performed by the cutting pedal 125.

Furthermore, in this embodiment, as shown in Figure 16, the first controller 310 is configured to limit the amount of movement of the servo motors SM2 to the predetermined threshold value TH1 or less when the gripping input is performed after the cutting procedure input is performed by the cutting pedal 125. As a result, even when the gripping input is performed after the procedure input is performed by the cutting pedal 125, the gripping force of the end effector 211 can be made low, thereby preventing a short circuit from occurring due to a strong gripping force.

Furthermore, in this embodiment, the first controller 310 is configured to release the limitation on the amount of movement of the servo motors SM2 to be equal to or less than the predetermined threshold value TH1 when the procedure input by the cutting pedal 125 is completed, and to terminate the transmission of the signal regarding electrical energy supply for cutting to the electrosurgical unit 220. As a result, after the surgical treatment by the surgical instrument 210 is completed, the cutting procedure input by the cutting pedal 125 is terminated, thereby releasing the limitation on the amount of movement of the servo motors SM2 to be equal to or less than the predetermined threshold value TH1, and the supply of electrical energy from the electrosurgical unit 220 to the surgical instrument 210 can be stopped. Also, by releasing the limitation on the amount of movement of the servo motors SM2 to be equal to or less than the predetermined threshold value TH1, when a surgical treatment by the surgical instrument 210 is not performed or when coagulation is performed as a surgical treatment, the gripping force of the end effector 211 can be made stronger than when the amount of movement of the servo motors SM2 is limited to or less than the predetermined threshold value TH1, and a procedure requiring a strong gripping force can be performed.

The first controller 310 is also configured to limit the amount of movement of the servo motors SM2 to within a predetermined range that is less than the predetermined threshold TH1 and greater than or equal to a predetermined threshold TH2 (TH2<TH1). The first controller 310 is configured to transmit the signal regarding electrical energy supply to the electrosurgical unit 220 based on the amount of movement of the servo motor SM2 falling within the predetermined range. The predetermined threshold TH1 is set, for example, to a value smaller than the maximum amount of movement of the servo motors SM2 when no procedure input is being performed by the cutting pedal 125. The predetermined threshold TH2 is set, for example, to the amount of movement of the servo motors SM2 when no gripping input is being performed by the operation handle 111 (for example, zero).

FIG. 15 shows an example in which the procedure input is made by the cutting pedal 125 after the gripping input is made by the operation handle 111. In the example shown in FIG. 15, at time T1, the operator starts an operation to close the pair of grip members 111a of the operation handle 111. Then, as the pair of grip members 111a of the operation handle 111 are gradually closed during the period from time T1 to time T2, the grip input by the operation handle 111 is increased and the grip force of the end effector 211 (the operation amount of the servo motors SM2) is increased. Then, at time T3, the operator steps on the cutting pedal 125, and the procedure input by the cutting pedal 125 is started. Then, the movement amount of the servo motors SM2 is limited to the predetermined range that is equal to or less than the predetermined threshold value TH1 and equal to or more than the predetermined threshold value TH2. At time T3, the amount of movement of the servo motors SM2 is greater than the predetermined threshold TH1, so the servo motors SM2 are controlled so that the amount of movement of servo motors SM2 is reduced to or below the predetermined threshold TH1. As a result, the object is gripped by the end effector 211 with the limited gripping force. Then, at time T4, when the amount of movement of the servo motor SM2 reaches the predetermined threshold TH1, transmission of the ON signal to the electrosurgical unit 220 begins as the signal regarding electrical energy supply for cutting. Then, while the ON signal is being received by the electrosurgical unit 220, electrical energy for cutting is supplied from the electrosurgical unit 220 to the surgical instrument 210. Then, at time T5, when the operator releases the operation of stepping on the cutting pedal 125, the procedure input by the cutting pedal 125 is terminated. Then, the state in which the amount of movement of servo motors SM2 was limited to the predetermined range is released, and transmission of the ON signal to the electrosurgical unit 220 is terminated.

FIG. 16 shows an example in which the gripping input is made by the operation handle 111 after the procedure input is made by the cutting pedal 125. In the example shown in FIG. 16, at time T11, the operator steps on the cutting pedal 125. Then, the procedure input by the cutting pedal 125 is started. The amount of movement of the servo motors SM2 is limited to the predetermined range that is equal to or less than the predetermined threshold TH1 and equal to or greater than the predetermined threshold TH2. Since the amount of movement of the servo motors SM2 is equal to or greater than the predetermined threshold TH2 at time T11, at time T12 immediately after time T11, transmission of the ON signal is started as the signal regarding electrical energy supply for cutting to the electrosurgical unit 220. Then, while the ON signal is being received by the electrosurgical unit 220, electrical energy for cutting is supplied from the electrosurgical unit 220 to the surgical instrument 210. Then, at time T13, the operator starts closing the pair of grip members 111a of the operation handle 111. Then, from time T13 to time T14, when the pair of grip members 111a of the operation handle 111 are gradually closed, the gripping input by the operation handle 111 is increased and the gripping force of the end effector 211 (amount of movement of the servo motors SM2) is increased. At this time, because the amount of movement of the servo motors SM2 is limited, the object is gripped by the end effector 211 with the limited gripping force. Then, at time T15, when the operator releases the operation of stepping on the cutting pedal 125, the procedure input by the cutting pedal 125 is terminated. Then, the state in which the amount of movement of the servo motors SM2 was limited to the predetermined range is released, and the transmission of the ON signal to the electrosurgical unit 220 is terminated.

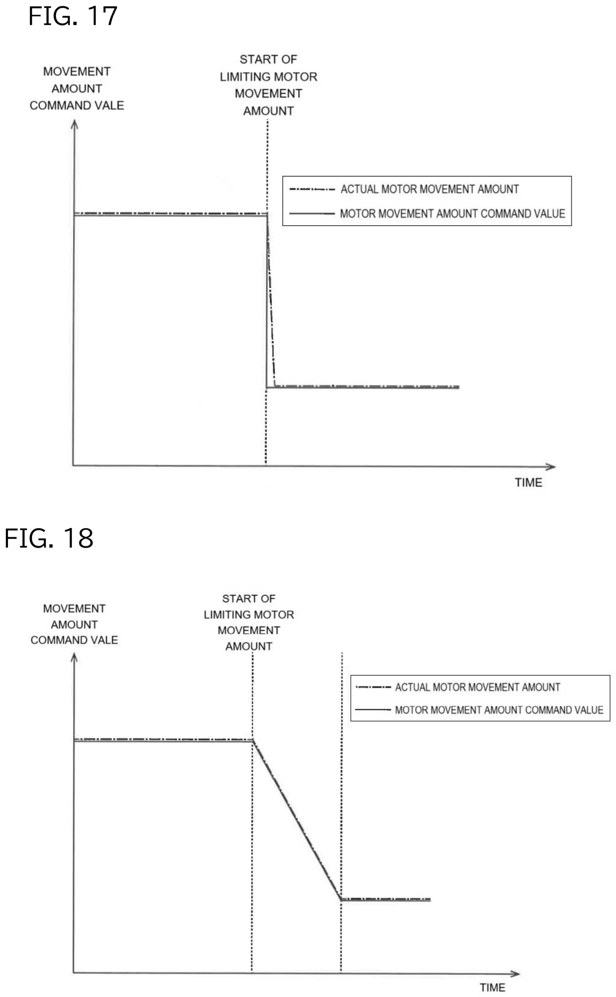

Here, when the amount of movement of the servo motors SM2 is reduced from a state greater than the predetermined threshold TH1 to a value equal to or less than the predetermined threshold TH1, if the servo motor SM2 is operated in response to a command to reduce the amount of movement of the servo motors SM2, the end effector 211 may move unnaturally (swaying by about 10 degrees) as a reaction. This is thought to be because, as in the example shown in FIG. 17, when the command value for the amount of movement of the servo motors SM2 is changed in an instant, there is a moment when there is a difference between the command value for the amount of movement of the servo motors SM2 and the actual amount of movement of the servo motor SM2, and the repulsive force causes the end effector 211 to move unnaturally.

Therefore, in this embodiment, as shown in FIG. 18, when the procedure input is performed by the cutting pedal 125 in a state where the amount of movement of the servo motors SM2 is greater than the predetermined threshold TH1 due to the gripping input, the first controller 310 controls the servo motors SM2 to reduce the amount of movement of the servo motors SM2 to less than the predetermined threshold TH1 at a speed slower than a maximum speed of the servo motors SM2. This allows the command value for the amount of movement of the servo motors SM2 to be changed more gradually than when the amount of movement of the servo motors SM2 is reduced to less than the predetermined threshold TH1 at the maximum speed of the servo motors SM2, making it possible to suppress the difference between the command value for the amount of movement of the servo motors SM2 and the actual amount of movement of the servo motors SM2. As a result, it is possible to suppress unnatural movement of the end effector 211 due to the difference between the command value for the amount of movement of the servo motors SM2 and the actual amount of movement of the servo motors SM2.

Furthermore, in this embodiment, when the procedure input is performed using the cutting pedal 125 in a state where the gripping input causes the amount of movement to be greater than the predetermined threshold TH1, the first controller 310 controls the servo motors SM2 to reduce the amount of movement of the servo motors SM2 to below the predetermined threshold TH1 over a predetermined time. This allows the command value for the amount of movement of the servo motors SM2 to be changed more gradually than when the command value for the amount of movement of the servo motors SM2 is changed instantly, thereby preventing the difference between the command value for the amount of movement of the servo motors SM2 and the actual amount of movement of the servo motors SM2. As a result, it is possible to prevent the end effector 211 from moving unnaturally due to the difference between the command value for the amount of movement of the servo motors SM2 and the actual amount of movement of the servo motors SM2.

For example, the first controller 310 may control the servo motors SM2 so that the movement amount of the servo motors SM2 reaches the predetermined threshold value TH1 or less in a fixed time. Further, for example, the first controller 310 may control the servo motors SM2 so that the movement amount of the servo motor SM2 reaches the predetermined threshold value TH1 or less while changing the movement amount at a fixed rate of change per unit time. Further, for example, the first control device 310 may control the servo motors SM2 so that the operation amount of the servo motors SM2 reaches a predetermined threshold value TH1 or less at a fixed servo motors SM2 speed (such as a speed that is a predetermined percentage of the maximum speed). This makes it possible to easily and gradually change the command value for the operation amount of the servo motors SM2.

### (Modifications)

Note that one or more embodiments disclosed herein should be considered as exemplary in all respects and do not limit the invention. The scope of the invention is indicated by claims, not by explanation of one or more embodiments described above, and includes equivalents to the claims and all alterations (modification) within the same.

In the above embodiment described above, the first controller 310 and the third controller 300 are separate control devices, but the invention is not limited to this. For example, the first controller 310 and the third controller 300 may be configured as a single control device.

In the above embodiment, the first controller 310 is configured to limit the amount of movement of the servo motor SM2 to the predetermined threshold value TH1 or less by changing the relationship between the opening/closing angle of the operation handle 111 and the opening/closing angle of the end effector 211, but the invention is not limited to this. For example, in a configuration in which the amount of movement of the servo motors SM2 is controlled based on a relationship between the opening/closing angle of the operation handle 111 and values of the current supplied to the servo motors SM2, the first controller 310 may be configured to limit the amount of movement of the servo motors SM2 to the predetermined threshold value TH1 or less by changing the relationship between the opening/closing angle of the operation handle 111 and the values of the current supplied to the servo motors SM2. In this way, the amount of movement of the servo motors SM2 can be limited to the predetermined threshold value TH1 or less simply by changing the relationship between the opening/closing angle of the operating handle 111 and the values of the current supplied to the servo motors SM2.

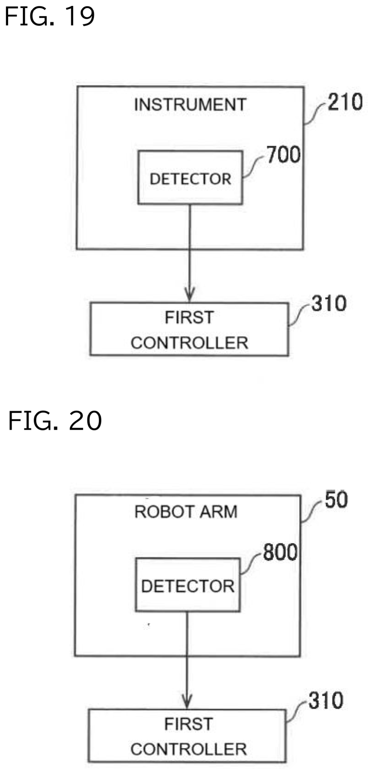

Also, for example, in the modified example shown in FIG. 19, the surgical instrument 210 is equipped with a detector 700 that detects the gripping force of the end effector 211. In this case, the first controller 310 may be configured to limit the amount of movement of the servo motors SM2 to the predetermined threshold value TH1 or less based on the detection value of the detector 700. As a result, the amount of movement of the servo motors SM2 can be limited to the predetermined threshold value TH1 or less based on the gripping force of the end effector 211 detected by the detector 700, so that the gripping force of the end effector 211 can be easily and accurately reduced to the desired gripping force.

Also, for example, in the modified example shown in FIG. 20, the robot arm 50 is equipped with a detector 800 that detects the torque of the servo motor SM2. In this case, the first controller 310 may be configured to limit the amount of movement of the servo motors SM2 to the predetermined threshold value TH1 or less based on the detection value of the detector unit 800. As a result, the amount of movement of the servo motors SM2 can be limited to the predetermined threshold value or less based on the torque of the servo motors SM2, which correlates with the gripping force of the end effector 211, so that the gripping force of the end effector 211 can be easily and accurately reduced to the desired gripping force.

In the above embodiment, the third controller 300 is configured to transmit the ON signal to the electrosurgical unit 220 as the signal regarding electrical energy supply, but the invention is not limited to this. In the invention, when the third controller 300 and the electrosurgical unit 220 are configured to be able to communicate information with each other, the third controller 300 may be configured to transmit a control signal for supplying electrical energy to the electrosurgical unit 220 as the signal regarding electrical energy supply.

In the above embodiment, the servo motor SM2 is provided as the actuator that drives the end effector 211 of the surgical instrument 210, but the invention is not limited to this. For example, a motor other than the servo motor may be provided as the actuator that drives the end effector 211 of the surgical instrument 210, or an actuator other than a motor may be provided.

In the above embodiment, the procedure input was performed using a foot pedal (cutting pedal 125), but the invention is not limited to this. For example, a switch for performing the procedure input may be provided on the grip member 111a or the grip support member 111b of the operation handle 111.

In the above embodiment, the arm portion 51 and the positioner 30 were configured as the 7-axis multi-joint robot, but the invention is not limited to this. For example, the arm portion 51 and the positioner 30 may be configured as the multi-joint robot with an axis configuration other than the 7-axis multi-joint robot. An axis configuration other than the 7-axis multi-joint robot is, for example, a 6-axis or 8-axis configuration.

In the above embodiment, the surgical robot 100 includes the medical cart 10, the positioner 30, and the arm base 40, but the invention is not limited to this. For example, the positioner 30 and the arm base 40 are not necessarily required, and the surgical robot 100 may be composed of the medical cart 10 and the robot arm 50. In this case, three surgical robots 100 (one for endoscopes and two for surgical instruments) or four surgical robots 100 (one for endoscopes and three for surgical instruments) can be used.

## Claims

1. A robotic surgical system (500) comprising:
a bipolar electrosurgical instrument (210) including a gripper (211a, 211b) that is able to be opened and closed to grip an object;
a robot arm (50) to which the electrosurgical instrument is detachably attached and which includes an actuator (SM2) configured to drive the gripper of the electrosurgical instrument;
a first input device (111) including a pair of grip members (111a) that are able to be opened and closed and receiving a gripping input instructing gripping by the gripper;
a second input device (125) that receives a cutting procedure input instructing to perform cutting as a surgical procedure by the electrosurgical instrument; and
one or more control devices (300, 310) configured to control an amount of movement of the actuator so that the gripper closes in response to the gripping input received by the first input device, wherein
the one or more control devices are configured to limit the amount of movement of the actuator for closing the gripper corresponding to the gripping input to a predetermined threshold (TH1) or less when the cutting procedure input is received by the second input device.

2. The robotic surgical system according to claim 1, wherein
the one or more control devices are configured to limit the amount of movement of the actuator to the predetermined threshold or less by changing a relationship between an opening/closing angle of the grip members and an opening/closing angle of the gripper, or a relationship between an opening/closing angle of the grip members and a current value supplied to the actuator.

3. The robotic surgical system according to claim 1, wherein
the electrosurgical instrument includes a detector (700) that detects a gripping force of the gripper, and
the one or more control devices are configured to limit the amount of movement of the actuator to the predetermined threshold or less based on a detection value of the detector.

4. The robotic surgical system according to claim 1, wherein
the robot arm includes a detector (800) that detects a torque of the actuator, and
the one or more control devices are configured to limit the amount of movement of the actuator to the predetermined threshold or less based on a detection value of the detector.

5. The robotic surgical system according to any one of claims 1 to 4, wherein
the actuator is a motor (SM2), and
the amount of movement of the actuator is an amount of rotation of the motor.

6. The robotic surgical system according to any one of claims 1 to 5, wherein
when the cutting procedure input is received by the second input device in a state in which the amount of movement is greater than the predetermined threshold by received the gripping input, the one or more control devices are configured to control the actuator to reduce the amount of movement to the predetermined threshold or less at a speed lower than a maximum speed of the actuator.

7. The robotic surgical system according to any one of claims 1 to 5, wherein
when the cutting procedure input is received by the second input device in a state in which the amount of movement is greater than the predetermined threshold by received the gripping input, the one or more control devices are configured to control the actuator to reduce the amount of movement to the predetermined threshold or less over a predetermined time.

8. The robotic surgical system according to any one of claims 1 to 5, wherein
the one or more control devices are configured to limit the amount of movement of the actuator corresponding to the gripping input to the predetermined threshold or less when the gripping input is received after the cutting procedure input is received by the second input device.

9. The robotic surgical system according to any one of claims 1 to 8, further comprising an electrosurgical unit (220) that supplies electrical energy to the electrosurgical instrument, wherein
the one or more control devices are configured to transmits a signal regarding electrical energy supply to the electrosurgical unit while the cutting procedure input is received by the second input device, and
the electrosurgical unit is configured to supply the electrical energy to the electrosurgical instrument while the signal regarding electrical energy supply is received.

10. The robotic surgical system according to claim 9, wherein
when the cutting procedure input received by the second input device is terminated, the one or more control devices are configured to release the limitation of the amount of movement of the actuator, and to terminate transmitting of the signal regarding electrical energy supply to the electrosurgical unit.

11. The robotic surgical system according to claim 9 or 10, wherein
the one or more control devices are configured to transmit signal regarding electrical energy supply to the electrosurgical unit in response to the amount of movement becoming the predetermined threshold or less.

12. The robotic surgical system according to any one of claims 1 to 11, further comprising:
a remote operating apparatus (200) including the first input device and the second input device.

13. The robotic surgical system according to any one of claims 1 to 12, wherein
the gripper includes first and second jaw members (211a, 211b) that are openable and closable to each other, or includes a second jaw member that is openable and closable relative to a fixed first jaw member.

14. The robotic surgical system according to any one of claims 1 to 13, wherein
the one or more control devices are configured to control the amount of movement of the actuator so that the angles of the pair of grip members and the angles of the gripper have a predetermined relationship.

15. A computer-implemented method for controlling a robotic surgical system (500), the method comprising:
receiving, via a first input device (111a) provided in the robotic surgical system and including a pair of grip members (111a) that are able to be opened and closed, a gripping input instructing to grip the object by a gripper (211a, 211b) that is included in a bipolar electrosurgical instrument and is able to be opened and closed to grip an object;
in response to receiving the gripping input, controlling an amount of movement of an actuator (SM2) configured to drive the gripper so that the gripper closes, the actuator provided in a robot arm (50) to which the electrosurgical instrument is detachably attached;
receiving, via a second input device (125) provided in the robotic surgical system, a cutting procedure input instructing to perform cutting as a surgical procedure by the electrosurgical instrument; and
in response to receiving the cutting procedure input, limiting the amount of movement of the actuator for closing the gripper corresponding to the gripping input to a predetermined threshold (TH1) or less.
